(19) Europäisches Patentamt / European Patent Office / Office européen des brevets

(11) **EP 4 455 662 A1**

(12) **EUROPEAN PATENT APPLICATION**
published in accordance with Art. 153(4) EPC

(43) Date of publication:
**30.10.2024 Bulletin 2024/44**

(21) Application number: **22912029.0**

(22) Date of filing: **23.12.2022**

(51) International Patent Classification (IPC):
*G01N 33/483* (2006.01)     *G01N 21/31* (2006.01)
*C12P 7/62* (2022.01)     *G01N 21/17* (2006.01)

(52) Cooperative Patent Classification (CPC):
**C12P 7/62; G01N 21/17; G01N 21/31;
G01N 33/483**

(86) International application number:
**PCT/KR2022/021206**

(87) International publication number:
**WO 2023/121396 (29.06.2023 Gazette 2023/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **24.12.2021 KR 20210187341**

(71) Applicant: **CJ CheilJedang Corporation
Seoul 04560 (KR)**

(72) Inventors:
• **KIM, Da Eun
  Seoul 04560 (KR)**
• **SEO, Dong June
  Seoul 04560 (KR)**
• **PARK, Joo Hyun
  Seoul 04560 (KR)**
• **KIM, Ka-Yeong
  Seoul 04560 (KR)**

(74) Representative: **Dehns
St. Bride's House
10 Salisbury Square
London EC4Y 8JD (GB)**

(54) **CELL LYSIS MONITORING METHOD, AND METHOD FOR PRODUCING POLYHYDROXYALKANOATES BY USING SAME**

(57) The present invention relates to a method for producing polyhydroxyalkanoates (PHAs), and a cell crushing monitoring method, and provides a method for producing polyhydroxyalkanoates (PHAs), the method allowing the length and sphericity of cells or PHAs to be measured through three-dimensional image analysis using the difference in refractive index of cells or PHAs, so that the autolysis degree, feed status, or crushing degree of cells of PHAs is identified, and thus optimum crushing conditions are set and the stability of an impurity removal process, which is a back-end process of a crushing process, can be increased.

[Fig. 1]

Culture medium preparation step → Analysis step → Crushing step → Analysis step

Feed front     Feed back

## Description

### Technical Field

The present invention relates to a method for monitoring cell crush and to a method for producing a polyhydroxyalkanoate (PHA) using the same.

### Background Art

Polyhydroxyalkanoates (PHAs) are biodegradable polymers composed of several types of hydroxyl carboxylic acids produced by numerous microorganisms and used as intracellular storage materials. Polyhydroxyalkanoates have physical properties similar to those of conventional petroleum-derived synthetic polymers such as polybutylene adipate terephthalate (PBAT), polybutylene succinate (PBS), polybutylene succinate terephthalate (PBST), and polybutylene succinate adipate (PBSA), exhibit complete biodegradability, and are excellent in biocompatibility.

Since PHA has the characteristic of forming and accumulating granules inside microbial cells, the most important step in the preparation and utilization processes of PHA is a step of extracting PHA granules from inside the cells through cell crush.

This extraction method of PHA granules may be mainly classified into three types: organic solvent extraction method, chemical extraction method, and physical crushing method.

First, the organic solvent extraction method is a method of dissolving and extracting PHA granules using an organic solvent. Although it can obtain high-purity PHA, it has the disadvantage of requiring dedicated equipment for the use of organic solvents. On the other hand, the chemical extraction method extracts PHA granules by disrupting the cell walls at high temperatures under acidic or basic conditions. Although the recovery of PHA granules is easy, problems may arise, such as a decrease in the molecular weight of the extracted PHA due to harsh acid or base conditions and temperature conditions.

Meanwhile, the physical crushing method is a method of extracting PHA granules after disrupting the cell walls with physical energy such as high pressure. It has the disadvantage of low purity of the extracted PHA granules due to its lower ability to remove impurities attached to the PHA granules as compared with the above methods. However, it has the advantage of minimizing a decrease in the molecular weight and the degradation of PHA: for, they are extracted under mild conditions. This physical crushing method includes ultrasonic crushing, high-pressure crushing, or milling crushing. High-pressure crushing includes cell crushing using a high-pressure homogenizer (HPH), and milling crushing includes a colloid mill, a bead mill, and a ball mill.

However, in the physical crushing method, the degree of cell crush may vary depending on the state of cells (size, PHA content in the cells, cell wall thickness and components, and the like) even when equal physical energy is applied. Thus, for process optimization and stabilization, quantitative and qualitative monitoring of cell status in real time is required before, during, or after the cell crushing step.

Specifically, conventional crush indicators used in physical crushing methods include optical microscopy, measurement of protein content, measurement of changes in target material concentration, dielectric spectroscopy, flow cytometry, confirmation of particle size distribution, SDS-page, and the like. They have disadvantages in that the analysis is complicated, the analysis time is long, and sample pretreatment is necessary.

The present inventors have devised the present invention to solve the problems of the prior art, which fails to allow an immediate change of process conditions according to the degree of cell crush during physical crushing using a high-pressure homogenizer, and to provide an effective crush indicator method.

[Prior Art Document]

[Non-patent Document]

(Non-patent Document 1) Britta, Eggenreich, "A combination of HPLC and automated data analysis for monitoring the efficiency of high-pressure homogenization," Microbial Cell Factories, Volume 16, Article No.: 134 (2017)

### Detailed Description of the Invention

### Technical Problem

The present invention provides a method for monitoring cell crush to determine the degree of cell crush by measuring cell length and cell sphericity through three-dimensional image analysis using differences in the refractive index of cells; and a method for producing a polyhydroxyalkanoate (PHA) using the same to set optimal cell crushing conditions and increase the stability of an impurity removal step subsequent to the crushing step.

**Solution to the Problem**

According to an aspect of the present invention, there is provided a method for monitoring cell crush, wherein the degree of cell crush is confirmed, or the crushing conditions are determined by analyzing a morphological parameter of cells or a polyhydroxyalkanoate (PHA) contained in a culture medium containing the PHA through three-dimensional image analysis.

In an embodiment, there is provided a method for monitoring cell crush in which the three-dimensional image analysis is a three-dimensional image analysis using holotomography analysis, and the morphological parameter is one or more selected from the group consisting of length, sphericity, volume, and surface area.

According to another aspect of the present invention, there is provided a method for producing a polyhydroxyalkanoate (PHA) that comprises preparing a culture medium containing a polyhydroxyalkanoate (PHA); preparing a feed solution containing the culture medium (or a supernatant obtained by centrifuging the culture medium); crushing the cells or PHA contained in the feed solution; and analyzing a morphological parameter of the cells or PHA before, during, or after the crushing step.

In an embodiment, analyzing a morphological parameter of the cells or PHA contained in the culture medium is carried out before the crushing step, and the method comprises quantifying the analyzed morphological parameter to detect a degree of autolysis; and determining the initially set pressure of the high-pressure homogenizer or the number of crushing treatments according to the degree of autolysis as a first pre-determination step of crushing conditions.

In another embodiment, analyzing a morphological parameter of the cells or PHA contained in the feed solution is carried out before the crushing step, and the method comprises quantifying the analyzed morphological parameter to detect a feed status; and determining the crushing pressure of the high-pressure homogenizer or the number of crushing treatments according to the feed status as a second pre-determination step of crushing conditions.

In another embodiment, analyzing a morphological parameter of the cells or PHA contained in the crushed product solution is carried out during, or after, the crushing step, and the method comprises quantifying the analyzed morphological parameter to detect a degree of crush; and determining the crushing pressure of the high-pressure homogenizer or the number of crushing treatments according to the degree of crush as a first re-determination step of crushing conditions.

In another embodiment, the crushing pressure in the crushing step is 300 to 1,500 bar.

In another embodiment, the method comprises preparing a culture medium containing a polyhydroxyalkanoate (PHA); preparing a feed solution containing the culture medium (or a supernatant obtained by centrifuging the culture medium); a first analysis step of analyzing a morphological parameter of the cells or PHA contained in the feed solution; a second pre-determination step of crushing conditions to determine the crushing pressure of the high-pressure homogenizer or the number of crushing treatments according to the first analysis results; a step of crushing the cells or PHA contained in the feed solution; a second analysis step of analyzing a morphological parameter of the cells or PHA contained in the crushed product solution; and a first re-determination step of crushing conditions to determine the crushing pressure of the high-pressure homogenizer or the number of crushing treatments according to the second analysis results.

In another embodiment, the degree of protein solubilization according to the following Equation 1 is 75 to 110%.

[Equation 1]

$$\text{Degree of protein solubilization (\%)} = ABS1/ABS0 \times 100 \, (\%)$$

In Equation 1, ABS0 is the absorbance at a wavelength of 280 nm measured after 5 times repeatedly crushing the culture medium, feed solution, or crushed product solution at 800 bar at room temperature; and ABS 1 is the absorbance at a wavelength of 280 nm measured after 1 time crushing the culture medium, feed solution, or crushed product solution at 300 to 1,500 bar at room temperature.

In another embodiment, when the average sphericity of the cells or PHA contained in the feed solution is 0.7 or more, or when the degree of protein solubilization according to the Equation 1 is 80% or more, in the second pre-determination step of crushing conditions the pressure is reduced by 10 to 100 bar from the initial set pressure in the step of crushing the cells or PHA contained in the feed solution.

In another embodiment, when the average sphericity of the cells or PHA contained in the crushed product solution is less than 0.9, or when the degree of protein solubilization according to the Equation 1 is less than 90%, in the first re-determination step of crushing conditions the pressure is raised by 10 to 100 bar from the crushing pressure in the step of crushing the cells or PHA contained in the feed solution.

In another embodiment, the method further comprises carrying out an agglomeration test to measure the degree of agglomeration of the crushed product solution after the second analysis step is carried out; and as a second re-determination step of crushing conditions determining the crushing pressure of the high-pressure homogenizer or the number of crushing treatments according to the degree of agglomeration.

According to another aspect of the present invention, there is provided a system for monitoring cell crush in which the degree of cell crush is confirmed, or the crushing conditions are determined by analyzing a morphological parameter of cells or a polyhydroxyalkanoate (PHA) contained in a culture medium containing the PHA through three-dimensional image analysis.

## Advantageous Effects of the Invention

The monitoring method according to an embodiment of the present invention enables accurate and real-time determination of the status of cells or a PHA even when a simple measurement method and a small amount of analysis sample are used, and it enables visualization of cell crush by implementing 3D stereoscopic photos. This allows an immediate change of the crushing process conditions depending on the status of the cells or PHA.

In addition, as the method for producing a polyhydroxyalkanoate (PHA) according to an embodiment of the present invention utilizes the above monitoring method, it is possible to immediately determine the status of cells or a PHA, allowing an immediate change of the crushing process conditions depending on the status of the cells or PHA. Specifically, the status of cells or a PHA contained in a culture medium is measured to set the crushing conditions in advance, and the feed status or degree of the crush of cells or a PHA is measured to change the crushing conditions in real time.

In addition, as the method for producing a polyhydroxyalkanoate (PHA) according to an embodiment of the present invention determines the optimal crushing pressure or the number of crushing treatments through the above monitoring method without additionally manipulating the temperature to high temperatures, it is possible to efficiently change the crushing conditions in real time.

As the method for producing a polyhydroxyalkanoate (PHA) according to an embodiment of the present invention measures the condition of cells or a PHA before, during, or after crushing to change the process conditions for crushing, separation, and impurity removal, it is possible to prevent the agglomeration of a PHA during the separation process and impurity removal process after the crushing process and to ensure the stability of the process.

## Brief Description of the Drawing

Fig. 1 is a simplified illustration of a method for producing a polyhydroxyalkanoate (PHA) according to an embodiment of the present invention.

Fig. 2 shows an algorithm for a method for producing a polyhydroxyalkanoate (PHA) according to an embodiment of the present invention.

Fig. 3 shows an example of an algorithm for controlling the crushing pressure in the crushing conditions pre-determination step.

Fig. 4 shows an example of an algorithm for controlling the crushing pressure in the crushing conditions re-determination step.

Fig. 5 shows another example of an algorithm for controlling the crushing pressure in the crushing conditions re-determination step.

Fig. 6 is a measurement result of an HTA image according to an embodiment.

Fig. 7 shows an example of a method for producing a polyhydroxyalkanoate of the present invention in which the risk areas in the separation process and impurity removal process according to cell length and sphericity are diagrammed when the culture medium of the example is used.

## Best Mode for Carrying out the Invention

Hereinafter, the present invention will be described in detail. The present invention is not limited to the disclosures given below, but it may be modified into various forms as long as the gist of the invention is not changed.

Throughout the present specification, when a part is referred to as "comprising" an element, it is understood that other elements may be comprised, rather than other elements are excluded, unless specifically stated otherwise.

All numbers and expressions related to the quantities of components, reaction conditions, and the like used herein are to be understood as being modified by the term "about," unless otherwise indicated.

In the present specification, in the case where an element is mentioned to be formed "on" or "under" another element, it means not only that one element is directly formed "on" or "under" another element, but also that one element is indirectly formed on or under another element with other element(s) interposed between them.

Throughout the present specification, the terms first, second, and the like are used to describe various components. But the components should not be limited by the terms. The terms are used only for the purpose of distinguishing one component from another.

### Method for monitoring cell crush and system for monitoring cell crush

In the method for monitoring cell crush according to an embodiment of the present invention, the degree of cell crush can be confirmed, or the crushing conditions can be determined by analyzing a morphological parameter of cells or a polyhydroxyalkanoate (PHA) contained in a culture medium containing the PHA through three-dimensional image analysis.

In the system for monitoring cell crush according to another embodiment of the present invention, the degree of cell crush can be confirmed, or the crushing conditions can be determined by analyzing a morphological parameter of cells or a polyhydroxyalkanoate (PHA) contained in a culture medium containing the PHA through three-dimensional image analysis.

The three-dimensional image analysis of an embodiment of the present invention may be a three-dimensional image analysis using holotomography analysis (HTA), and the morphological parameter is one or more types selected from the group consisting of length, sphericity, volume, and surface area.

The holotomography analysis is a laser technology that measures the three-dimensional refractive index of a microscopic sample such as cells and is a type of three-dimensional microscope. It can provide quantitative image information about the morphological parameters of cells through the measured refractive index. As a result, the length, sphericity, volume, surface area, density, or mass of cells can be calculated.

In addition, the holotomography analysis can clearly image cell membranes or intracellular organelles even without using exogenous labels; thus, there are no problems with phototoxicity, photobleaching, or photodamage. It is possible to visualize the degree of cell crush by achieving three-dimensional stereoscopic photos through holotomography analysis.

In addition, holotomography analysis provides a spatial resolution of approximately 100 nm and a temporal resolution of hundreds of frames per second depending on the number of apertures of the objective lens used and the resolution of the image sensor, enabling accurate measurement even with a small amount of sample of 10 $\mu$l of a diluent. Since the total measurement time is 10 minutes or less, quick measurement is possible. Thus, the status of cells can be checked in real time by using holotomography analysis. Thus, the crushing process conditions can be changed immediately or set in advance.

The holotomography analysis can be performed by diluting a sample to be analyzed 10 to 100 times using distilled water (DIW) and taking a three-dimensional image, and the refractive index (RI) used in taking the three-dimensional image may be 1.35 to 1.50 or 1.35 to 1.45, but they are not limited thereto.

The length, sphericity, volume, surface area, density, or mass of cells or a PHA can be calculated through the three-dimensional image analysis.

### Method for producing a polyhydroxyalkanoate (PHA)

The method for producing a polyhydroxyalkanoate (PHA) according to an embodiment of the present invention comprises preparing a culture medium containing a polyhydroxyalkanoate (PHA) (culture medium preparation step); preparing a feed solution containing the culture medium (or a supernatant obtained by centrifuging the culture medium) (feed solution preparation step); crushing the cells or PHA contained in the feed solution (crushing step); and analyzing a morphological parameter of the cells or PHA before, during, or after the crushing step (analysis step).

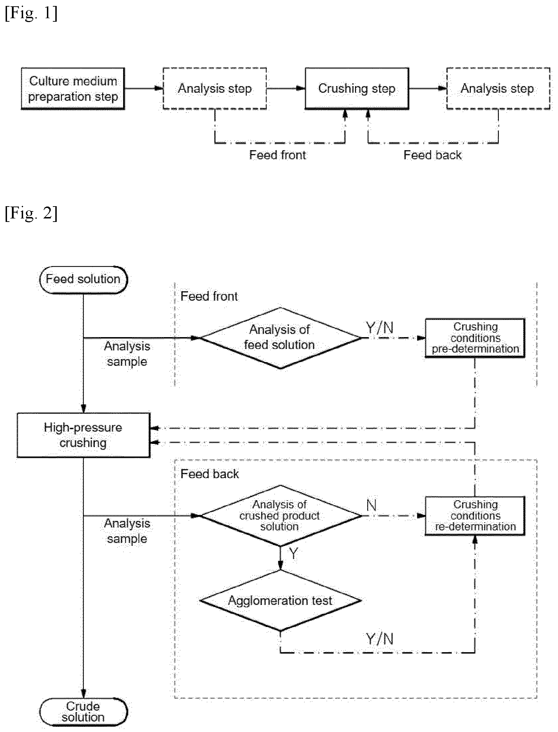

The method for producing a polyhydroxyalkanoate (PHA) that comprises the step of analyzing a morphological parameter of the cells or PHA before, during, and/or after the crushing step (analysis step) is shown in Fig. 1 or Fig. 2.

Cell crush can be carried out as a batch-type reaction or as a continuous production process. In a continuous production process, the morphological parameter of the cells or PHA is analyzed in real time, and then the crushing step can be carried out by adjusting the crushing conditions for the feed solution continuously fed in real time according to the analysis results.

The crushing conditions may be the same or different. The crushing step can be carried out by repeating the above procedure and re-determining the crushing conditions in real time.

In addition, the method for producing a polyhydroxyalkanoate (PHA) of the present invention may comprise analyzing a morphological parameter of the cells or PHA contained in the feed solution (first analysis step); and crushing the cells or PHA contained in the feed solution (crushing step).

In addition, in the method for producing a polyhydroxyalkanoate (PHA) of the present invention, analyzing a morphological parameter of the cells or PHA contained in the crushed product solution is carried out during, or after, the crushing step, and the method may comprise quantifying the analyzed morphological parameter to detect the degree of crush (second analysis step); and determining the crushing pressure of the high-pressure homogenizer or the number of crushing treatments according to the degree of crush as a first re-determination step of crushing conditions.

Specifically, the method for producing a polyhydroxyalkanoate (PHA) of the present invention may comprise preparing a culture medium containing a polyhydroxyalkanoate (PHA) (culture medium preparation step); analyzing a morphological parameter of the cells or PHA contained in the culture medium (preliminary analysis step); determining the initially set conditions of the culture medium according to the preliminary analysis results (first crushing conditions pre-determination step); preparing a feed solution containing the culture medium (or a supernatant obtained by centrifuging the culture medium) (feed solution preparation step); a first analysis step of analyzing a morphological parameter of the cells or PHA contained in the feed solution (first analysis step); determining the crushing pressure of the high-pressure homogenizer or the number of crushing treatments according to the first analysis results (second crushing conditions pre-determination step); crushing the cells or PHA contained in the feed solution (crushing step); a second analysis step of analyzing a morphological parameter of the cells or PHA contained in the crushed product solution (second analysis step); determining the crushing pressure of the high-pressure homogenizer or the number of crushing treatments according to the second analysis results (first crushing conditions re-determination step); carrying out an agglomeration test to measure the degree of agglomeration of the crushed product solution (agglomeration test step); and/or determining the crushing pressure of the high-pressure homogenizer or the number of crushing treatments according to the degree of agglomeration (second crushing conditions re-determination step).

The preliminary analysis step, first crushing conditions pre-determination step, first analysis step, second crushing conditions pre-determination step, second analysis step, first crushing conditions re-determination step, agglomeration test step, and second crushing conditions re-determination step are each independent and may be carried out selectively.

The method for producing a polyhydroxyalkanoate (PHA) according to an embodiment of the present invention may be carried out by the following exemplary method.

(1) Culture medium preparation step; preliminary analysis step; and crushing step
(2) Culture medium preparation step; crushing step; and second analysis step
(3) Culture medium preparation step; first analysis step; crushing step; and second analysis step
(4) Culture medium preparation step; first analysis step; crushing step; second analysis step; and first crushing conditions re-determination step
(5) Culture medium preparation step; feed solution preparation step; first analysis step; and crushing step
(6) Culture medium preparation step; preliminary analysis step; feed solution preparation step; first analysis step; and crushing step
(7) Culture medium preparation step; feed solution preparation step; first analysis step; second crushing conditions pre-determination step; and crushing step
(8) Culture medium preparation step; first analysis step; second crushing conditions pre-determination step; crushing step; second analysis step; and first crushing conditions re-determination step
(9) Culture medium preparation step; feed solution preparation step; first analysis step; second crushing conditions pre-determination step; crushing step; second analysis step; and first crushing conditions re-determination step
(10) Culture medium preparation step; preliminary analysis step; feed solution preparation step; first analysis step; second crushing conditions pre-determination step; crushing step; second analysis step; and first crushing conditions re-determination step

Further, the method for producing a polyhydroxyalkanoate (PHA) according to an embodiment of the present invention may be carried out by various steps in addition to the steps exemplified above. If necessary, the first analysis step, the first or second crushing conditions pre-determination step, the first or second crushing conditions re-determination step, and the crushing step may be repeatedly carried out.

Hereinafter, each step will be described in detail.

**Culture medium preparation step**

The method for producing a polyhydroxyalkanoate (PHA) according to an embodiment of the present invention comprises preparing a culture medium containing a polyhydroxyalkanoate (PHA).

In general, a polyhydroxyalkanoates can be produced by a microbial fermentation process. The crude product of a polyhydroxyalkanoate may also be obtained through fermentation.

The method for producing a polyhydroxyalkanoate (PHA) through fermentation may be carried out by a known method. For example, a polyhydroxyalkanoate can be produced through fermentation using wild-type or transgenic microorganisms cultured on a specific substrate, and it is not particularly limited. The microorganism may be a microorganism of the *Escherichia* genus or *Escherichia coli,* but it is not limited thereto.

The culture medium is cultured with various microorganisms capable of producing a polyhydroxyalkanoate (PHA). The culture medium may be prepared by a conventional method in which microorganisms are cultured at 15 to 50°C or 30 to 40°C for 1 to 160 hours using a bioreactor in a normal medium appropriately containing a carbon source, a nitrogen source, a phosphorus source, an inorganic compound, amino acids, and/or vitamins. Compounds such as sodium hydroxide, ammonium hydroxide, potassium hydroxide, ammonia, phosphoric acid, sulfuric acid, and the like may be added to the medium in an appropriate manner during the cultivation of the microorganisms to adjust the pH of the medium, but it is not limited thereto.

Examples of the carbon source include carbohydrates such as glucose, saccharose, lactose, fructose, sucrose, and maltose; sugar alcohols such as mannitol and sorbitol; organic acids such as pyruvic acid, lactic acid, and citric acid; and amino acids such as glutamic acid, methionine, and lysine. In addition, natural organic nutrient sources such as starch hydrolyzate, molasses, blackstrap molasses, rice bran, cassava, bagasse, and corn steep liquor may be used. Other carbon sources in appropriate amounts may be used in a variety of ways without limitation. These carbon sources may be used alone or in combination of two or more types, but they are not limited thereto.

The polyhydroxyalkanoate (PHA) may be formed by an enzyme-catalyzed polymerization of one or more monomer repeat units in living cells. It may be classified as crystalline, semicrystalline, or amorphous polyhydroxyalkanoate depending on its molecular structure.

Examples of repeat units that constitute the polyhydroxyalkanoate (PHA) include lactic acid, glycolic acid, 2-hydroxybutyrate (2-HB), 3-hydroxybutyrate (3-HB), 3-hydroxypropionate (3-HP), 3-hydroxyvalerate (3-HV), 3-hydroxyhexanoate (3-HH), 3-hydroxyheptanoate (3-HHep), 3-hydroxyoctanoate (3-HO), 3-hydroxynonanoate (3-HN), 3-hydroxydecanoate (3-HD), 3-hydroxydodecanoate (3-HDd), 4-hydroxybutyrate (4-HB), 4-hydroxyvalerate (4-HV), 5-hydroxyvalerate (5-HV), and 6-hydroxyhexanoate (6-HH). The PHA may comprise one or more repeat units selected from the above. For example, the PHA may be poly-3-hydroxybutyrate-co-4-hydroxybutyrate (hereinafter, referred to as 3HB-co-4HB).

The polyhydroxyalkanoate (PHA) may be a homopolymer or a copolymer. According to an embodiment, the polyhydroxyalkanoate may comprise a copolymer, specifically, a copolymer comprising two or more different repeat units with the different repeat units randomly distributed in the polymer chain.

In addition, the polyhydroxyalkanoate may comprise isomers. For example, the polyhydroxyalkanoate may comprise structural isomers, enantiomers, or geometric isomers. Specifically, the polyhydroxyalkanoate may comprise structural isomers.

As an example, the polyhydroxyalkanoate (PHA) may comprise a 4-hydroxybutyrate (4-HB) repeat unit. For example, the content of a 4-HB repeat unit in the polyhydroxyalkanoate may be 0.1% by weight or more, 1% by weight or more, 5% by weight or more, 10% by weight or more, 20% by weight or more, 30% by weight or more, 40% by weight or more, 50% by weight or more, or 60% by weight or more; 100% by weight or less, 99% by weight or less, 90% by weight or less, 80% by weight or less, 70% by weight or less, or 60% by weight or less; and 0.1 to 60% by weight, 0.1 to 55% by weight, 0.5 to 60% by weight, 0.5 to 55% by weight, 1 to 60% by weight, 1 to 55% by weight, 1 to 50% by weight, 2 to 55% by weight, 3 to 55% by weight, 3 to 50% by weight, 5 to 55% by weight, 5 to 50% by weight, 10 to 55% by weight, 10 to 50% by weight, or 1 to 40% by weight, based on the total weight of the polyhydroxyalkanoate. As the content of a 4-HB repeat unit satisfies the above range, a PHA finally prepared would have excellent biodegradability in soil and sea, and it is possible to further enhance thermal characteristics, productivity, processability, and the like without deterioration of mechanical properties.

In addition, the polyhydroxyalkanoate (PHA) may have a weight average molecular weight of 10,000 to 1,200,000 g/mole. For example, the weight average molecular weight of the PHA may be 10,000 to 1,200,000 g/mole, 50,000 to 1,200,000 g/mole, 100,000 to 1,000,000 g/mole, 100,000 to 900,000 g/mole, 200,000 to 800,000 g/mole, 10,000 to 600,000 g/mole, or 600,000 to 1,200,000 g/mole.

The microbial cells for producing the polyhydroxyalkanoate (PHA) may be cells in a normal state, but they may be in a state of autolysis, a state in which the cell walls are weakened or the cells burst due to overcultivation or a self-digestion reaction. In the method according to an embodiment of the present invention, the crushing step can be carried out effectively even when the cells are in a normal state or in an autolysis state, and the productivity of a PHA finally obtained can be enhanced.

**Feed solution preparation step**

The method for producing a polyhydroxyalkanoate (PHA) according to an embodiment of the present invention comprises preparing a feed solution containing the culture medium (or a supernatant obtained by centrifuging the culture medium).

The feed solution is one in which cells and a PHA are diluted with distilled water. It may further comprise a pH-adjusting agent and/or a surfactant.

When the feed solution contains a supernatant obtained by centrifuging the culture medium, the centrifugation may be carried out at a speed of 3,000 to 8,000 rpm, 4,000 to 6,000 rpm, or 4,000 to 5,000 rpm for 10 to 50 minutes, 20 to 40 minutes, or 25 to 35 minutes, but it is not limited thereto.

The cells and PHA of the feed solution may be mixed with distilled water such that the total solids content is 5 to 20% by weight, preferably, the total solids content is 10 to 15% by weight. When the total solids content satisfies the above content range, a crushing step can be carried out efficiently.

The feed solution may comprise a pH-adjusting agent. The pH-adjusting agent may be one or more basic solutions selected from the group consisting of sodium hydroxide, potassium hydroxide, lithium hydroxide, and sodium carbonate. In addition, the concentration of the basic solution is such that the basic solute is 20 to 60 w/w%, 30 to 50 w/w%, or 35 to 45 w/w% based on the total weight of the basic solution.

The pH of the feed solution comprising the pH-adjusting agent may be pH 8 to pH 13, or pH 9 to pH 12, preferably pH 10 to pH 11. When the pH of the feed solution is adjusted to the above range, a cell crushing step and subsequent steps can be more readily carried out.

The feed solution may comprise a surfactant. The surfactant may be one or more anionic surfactants selected from the group consisting of sodium laureth sulfate, sodium lauryl sulfate, dodecyl sodium sulfate, and dodecyl sodium benzene sulfonate.

The surfactant may be employed in an amount of 0.05 to 0.5% by weight, 0.1 to 0.2% by weight, preferably 0.13 to 0.15% by weight, based on the total weight of the feed solution. When the content of the surfactant is adjusted to the above range, a cell crushing step and subsequent steps can be more readily carried out.

**Step of crushing the cells or PHA (crushing step)**

The method for producing a polyhydroxyalkanoate (PHA) according to an embodiment of the present invention comprises crushing the cells or PHA contained in the feed solution.

The crushing step is a physical cell crushing step using a high-pressure homogenizer. The high-pressure homogenizer is a device that crushes cells by applying high pressure to the cells. The crushing pressure may be 300 to 1,500 bar, 300 to 600 bar, 300 to 500 bar, 400 to 1,000 bar, 400 to 700 bar, 400 to 600 bar, 400 to 500 bar, 600 to 1,000 bar, or 600 to 800 bar.

When the crushing pressure satisfies the above range, the target degree of cell crush can be reached, an appropriate viscosity range can be maintained, which allows subsequent steps to be readily carried out, and the productivity of a PHA can be increased.

When the lower limit of the crushing pressure is reached, a crude polyhydroxyalkanoate solution is obtained immediately after the crushing step without an analysis step of the crushed product solution and an agglomeration test step.

When the upper limit of the crushing pressure is reached, a crude polyhydroxyalkanoate solution is obtained after an analysis step of the crushed product solution is carried out without an agglomeration test step.

In addition, the crushing temperature may be 0 to 40°C, 5 to 35°C, 10 to 30°C, 20 to 40°C, or 23 to 35°C. To prevent a rise in temperature due to high-pressure crushing, cooling water is flowed to keep the temperature constant.

Crushing time may vary depending on the flow rate entering or exiting the high-pressure homogenizer or the throughput per hour.

The crushing step is carried out once on the feed solution that passes through the high-pressure homogenizer. If the crushing step is carried out by changing the crushing conditions one or more times through a crushing conditions pre-determination step and/or a crushing conditions re-determination step, the determined crushing conditions (pressure, etc.) may be the same or different.

The crushing conditions may be determined according to the analysis results of a morphological parameter. Specifically, the morphological parameter of cells or a PHA may be analyzed before, during, or after the crushing step, and the number and conditions of crushing may be determined according to the analysis results. Details thereof will be described below in the crushing conditions pre-determination step and the crushing conditions re-determination step.

**Step of centrifuging the cell-crushed product solution to obtain a crude polyhydroxyalkanoate solution (separation step)**

The method for producing a polyhydroxyalkanoate (PHA) according to an embodiment of the present invention comprises centrifuging the cell-crushed product solution to obtain a crude polyhydroxyalkanoate solution.

The step of preparing a crude polyhydroxyalkanoate solution is a step of centrifuging the cell-crushed product solution to separate impurities and the cells and PHA contained in the crushed product solution.

The centrifugation may be carried out at a speed of 10,000 to 20,000 rpm, 12,000 to 18,000 rpm, or 15,000 to 16,000 rpm for 10 to 30 minutes or 10 to 20 minutes. In addition, the centrifugation may be carried out at a temperature of 0 to 40°C, 5 to 35°C, 10 to 30°C, 20 to 40°C, or 23 to 35°C.

A crude polyhydroxyalkanoate solution containing the retentate formed through the centrifugation is obtained. The crude polyhydroxyalkanoate solution thus obtained is then subjected to a washing step and an impurity removal step through additional chemical or enzymatical treatment to be formulated.

**Step of analyzing a morphological parameter of the cells or PHA (analysis step)**

The method for producing a polyhydroxyalkanoate (PHA) according to an embodiment of the present invention comprises analyzing a morphological parameter of the cells or PHA.

The step of analyzing a morphological parameter may be three-dimensional image analysis using holotomography analysis, but it is not limited thereto. In addition, the morphological parameter may be one or more selected from the group consisting of length, sphericity, mass, protein concentration, volume, and surface area.

Details of the holotomography analysis (HTA) are as described above.

In an embodiment of the present invention, the step of analyzing a morphological parameter of the cells or PHA may comprise a preliminary analysis step and a first analysis step carried out before the crushing step; and/or a second analysis step carried out during or after the crushing step.

The analysis step may comprise a preliminary analysis step of collecting some analysis samples from the culture medium, feed solution, and/or crushed product solution, and analyzing a morphological parameter of the cells or PHA contained in the culture medium according to the analysis sample collection point; a first analysis step of analyzing a morphological parameter of the cells or PHA contained in the feed solution; and/or a second analysis step of analyzing a morphological parameter of the cells or PHA contained in the crushed product solution.

Each analysis step is a separate step and is selected individually as needed. When multiple analysis steps are adopted simultaneously in one process, each analysis step may be carried out in the following order: preliminary analysis step, first analysis step, second analysis step, and auxiliary analysis step.

1) Analysis of the cells or PHA contained in the culture medium (preliminary analysis step)

The preliminary analysis step is a step of analyzing a morphological parameter of the cells or PHA contained in the culture medium and quantifying the analyzed morphological parameter to detect the degree of autolysis.

The average sphericity of the cells contained in the culture medium may be 0.4 or more, 0.5 or more, 0.6 or more, or 0.7 or more. In addition, the average length of the cells may be 3.0 $\mu$m or more or 3.5 $\mu$m or more, and may be 6.0 $\mu$m or less, 5.0 $\mu$m or less, or 4.0 $\mu$m or less.

The average sphericity of the PHA contained in the culture medium may be 0.5 or more, 0.6 or more, 0.7 or more, or 0.8 or more.

As the degree of autolysis increases, the average length of the cells in the culture medium becomes shorter, and the average sphericity of the cells or PHA increases.

2) Analysis of the cells or PHA contained in the feed solution (first analysis step)

In an embodiment of the present invention, the step of analyzing a morphological parameter of the cells or PHA may comprise a first analysis step carried out before the crushing step. The first analysis step is a step of analyzing a morphological parameter of the cells or PHA contained in the feed solution and quantifying the analyzed morphological parameter to detect the feed status.

The average sphericity of the cells contained in the feed solution may be 0.6 or more, 0.65 or more, 0.7 or more, 0.75 or more, 0.8 or more, 0.85 or more, or 0.9 or more. In addition, the average length of the cells may be 3.28 $\mu$m or more, 3.4 $\mu$m or more, 3.6 $\mu$m or more, 3.8 $\mu$m or more, 4.0 $\mu$m or more, or 4.2 $\mu$m or more, and 6.0 $\mu$m or less, 5.5 $\mu$m or less, 5.0 $\mu$m or less, or 4.8 $\mu$m or less.

Specifically, the average length of the cells contained in the feed solution may be 3.28 $\mu$m to 4.8 $\mu$m, 3.28 $\mu$m to 4.2 $\mu$m, or 4.0 $\mu$m to 4.8 $\mu$m, and the average sphericity of the cells may be 0.6 to 0.99, 0.65 to 0.9, 0.7 to 0.9, 0.6 to 0.7, or 0.75 to 0.85.

More specifically, the cells contained in the feed solution may comprise cells having a sphericity of 0 to 0.8 at a proportion of 40 to 90%, 40 to 80%, 45 to 70%, 50 to 70%, 55 to 65%, or 60 to 65%. In addition, the cells may comprise cells having a sphericity of 0.8 to 1.0 at a proportion of 10 to 60%, 20 to 55%, 30 to 55%, 30 to 50%, or 35 to 45%.

Further, the average sphericity of the PHA contained in the feed solution may be 0.6 or more, 0.65 or more, 0.7 or more, 0.75 or more, 0.8 or more, 0.85 or more, or 0.9 or more.

In addition, the PHA may comprise a PHA having a sphericity of 0 to 0.8 at a proportion of 23% or more, 25% or more, 30% or more, 34% or more, 35% or more, 40% or more, 45% or more, 50% or more, 55% or more, 60% or more, or 65% or more, and it may comprise a PHA having a sphericity of 0.8 to 1.0 at a proportion of 77% or less, 75% or less, 70% or less, 66% or less, 65% or less, 60% or less, 55% or less, 50% or less, 45% or less, 40% or less, or 35% or less.

3) Analysis of the cells or PHA contained in the crushed product solution (second analysis step)

In an embodiment of the present invention, the step of analyzing a morphological parameter of the cells or PHA may comprise a second analysis step carried out during or after the crushing step. The second analysis step is a step of analyzing a morphological parameter of the cells or PHA contained in the crushed product solution and quantifying the analyzed morphological parameter to detect the degree of crush.

Final cells and a PHA contained in the crushed product solution thus prepared may be obtained through the method for producing a polyhydroxyalkanoate (PHA) according to an embodiment of the present invention.

The average sphericity of the cells contained in the crushed product solution may be 0.65 or more, 0.7 or more, 0.8 or more, or 0.9 or more. In addition, the average length of the cells may be 3.0 $\mu$m or more or 3.5 $\mu$m or more, and may be 4.5 $\mu$m or less or 4.0 $\mu$m or less.

Further, the average sphericity of the PHA contained in the crushed product solution may be 0.6 or more, 0.65 or more, 0.7 or more, 0.75 or more, 0.8 or more, 0.85 or more, or 0.9 or more. The PHA may comprise a PHA having a sphericity of 0.8 to 1.0 at a proportion of 34% or more, 35% or more, 40% or more, 45% or more, 50% or more, 55% or more, 60% or more, or 65% or more, and it may comprise a PHA having a sphericity of 0 to 0.8 at a proportion of 66% or less, 65% or less, 60% or less, 55% or less, 50% or less, 45% or less, 40% or less, or 35% or less.

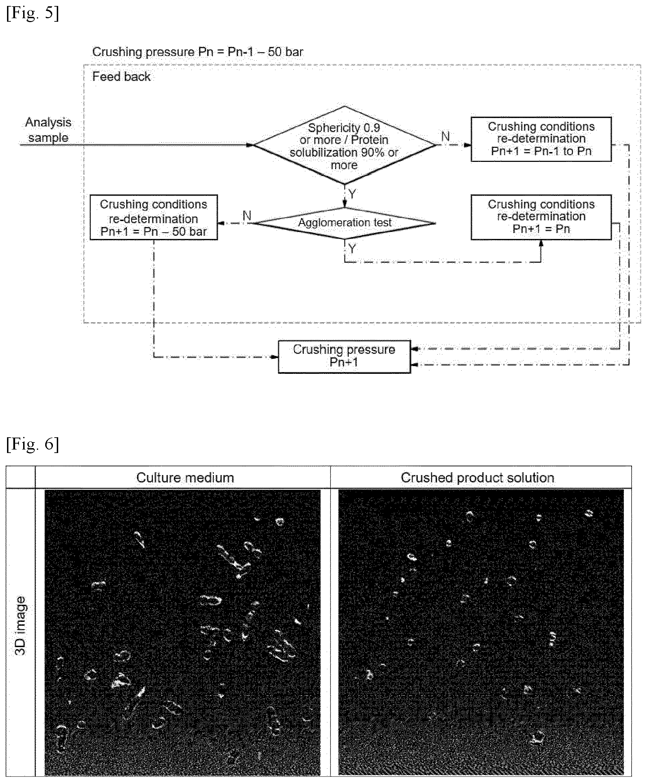

Three-dimensional images measured before and after cell crush are shown in Fig. 6. The bright parts (green) indicate cell walls or cytoplasm, and the dark parts (red) indicate a PHA. It can be visually confirmed through the three-dimensional images that, upon crush, the length of the cells or PHA has decreased, and the sphericity of the cells or PHA has increased.

**Step of analyzing the degree of protein solubilization or aggregation (auxiliary analysis step)**

1) Analysis of the degree of protein solubilization

Analysis of the degree of protein solubilization or an aggregation test may be carried out as an auxiliary indicator to determine whether the cells and PHA contained in the crushed product solution are sufficiently crushed to proceed with the subsequent steps and to ensure the stability of the process.

Specifically, the degree of protein solubilization is an auxiliary indicator to determine cell rupture and cell crush and may be calculated according to the following Equation 1 by measuring the absorbance at a wavelength of 280 nm with a UV-spectrometer.

[Equation 1]

$$\text{Degree of protein solubilization (\%)} = \text{ABS1/ABS0} \times 100 \text{ (\%)}$$

In Equation 1, ABS0 is the absorbance at a wavelength of 280 nm measured after 5 times repeatedly crushing the culture medium, feed solution, or crushed product solution at 800 bar at room temperature; and ABS 1 is the absorbance at a wavelength of 280 nm measured after 1 time crushing the culture medium, feed solution, or crushed product solution at 300 to 1,500 bar at room temperature. More specifically, ABS0 and ABS1 are each the absorbance measured by crushing the culture medium, feed solution, or crushed product solution under the above conditions, centrifuging it for 5 minutes at 15,000 rpm, and diluting the supernatant 100 times with distilled water.

In the present invention, room temperature refers to a constant temperature and may be a temperature of 10 to 40°C, 20 to 35°C, 23 to 35°C, or about 25°C. In the present invention, to prevent a rise in temperature due to high-pressure crushing, heat exchange is performed by flowing cooling water from the outside to keep constant the temperature of the apparatus such as a high-pressure homogenizer.

The degree of protein solubilization of the crushed product solution according to an embodiment of the present invention according to the above Equation 1 is 75 to 110%. Specifically, the degree of protein solubilization of the crushed product solution may be 85% or more, 90% or more, or 95% or more. If the degree of protein solubilization is less than 85%, the efficiency of cell crush decreases; thus, the efficiency of obtaining PHA granules in the PHA production method may decrease.

2) Agglomeration test

The agglomeration test may be used as an auxiliary indicator to confirm the stability of solid-liquid separation after cell crush. Whether the cells or PHA is agglomerated, and the degree of aggregation thereof, are measured by applying shear force or centrifugal force to the cell-crushed product solution after physical cell crushing using a high-pressure homogenizer, from which the stability of the subsequent washing step and impurity removal step can be confirmed.

In this test, the cell-crushed product solution is centrifuged at 3,800 rpm for 20 minutes, the supernatant is removed, and the crude polyhydroxyalkanoate solution is passed through a 150-$\mu$m sieve using washing water. In such an event, the degree of agglomeration can be checked from the residue that is not dissolved in the washing water and does not pass through the sieve. If the degree of agglomeration is high, agglomeration would be highly likely to occur during the subsequent washing step and impurity removal step. Thus, the production of a polyhydroxyalkanoate (PHA) is carried out by adjusting the crushing pressure or changing the process conditions of the washing step and impurity removal step.

**Crushing conditions pre-determination step**

In an embodiment of the present invention, analyzing a morphological parameter of the cells or PHA contained in the culture medium is carried out before the crushing step, and the method comprises quantifying the analyzed morphological parameter to detect the degree of autolysis (preliminary analysis step); and determining the initially set pressure of the high-pressure homogenizer or the number of crushing treatments according to the degree of autolysis as a first pre-determination step of crushing conditions.

In another embodiment of the present invention, analyzing a morphological parameter of the cells or PHA contained in the feed solution is carried out before the crushing step, and the method comprises quantifying the analyzed morphological parameter to detect the feed status (first analysis step); and determining the crushing pressure of the high-pressure homogenizer or the number of crushing treatments for the feed solution according to the feed status as a second pre-determination step of crushing conditions. Each crushing conditions pre-determination step is a separate step and is individually selected as needed. When the first pre-determination step and the second pre-determination step are adopted simultaneously in one process, the second pre-determination step is carried out after the first pre-determination step.

1) First crushing conditions pre-determination step according to the analysis of a morphological parameter
As an example, when the average sphericity of the cells or PHA analyzed in the step of analyzing a morphological parameter of the cells or PHA contained in the culture medium is 0.7 or more, the initially set pressure may be determined to be 700 bar or less or 600 bar or less.
The phenomenon of PHA agglomeration may occur during the centrifugation and impurity removal steps after cell crush depending on the degree of cell autolysis. Therefore, piping clogging or the like can be prevented in the subsequent steps to the crushing step through the analysis of a morphological parameter and adjustment of crushing conditions.
2) Second crushing conditions pre-determination step according to the analysis of a morphological parameter
As an example, when the average sphericity of the cells or PHA contained in the feed solution is 0.7 or more, the second crushing conditions pre-determination step may reduce the pressure by 10 to 100 bar from the initially set pressure in the step of crushing the cells or PHA contained in the feed solution.
As another example, when the average sphericity of the cells or PHA contained in the feed solution is less than 0.7, the second crushing conditions pre-determination step may determine the crushing pressure in the step of crushing the cells or PHA contained in the feed solution to be the same as the initially set pressure.
3) Second crushing conditions pre-determination step according to the analysis of the degree of protein solubilization

As an example, when the degree of protein solubilization of the feed solution according to the above Equation 1 is 80% or more, the second crushing conditions pre-determination step may reduce the pressure by 10 to 100 bar from the initially set pressure in the step of crushing the cells or PHA contained in the feed solution.

As another example, when the degree of protein solubilization of the feed solution according to the above Equation 1 is less than 80%, the second crushing conditions pre-determination step may determine the crushing pressure in the step of crushing the cells or PHA contained in the feed solution to be the same as the initially set pressure.

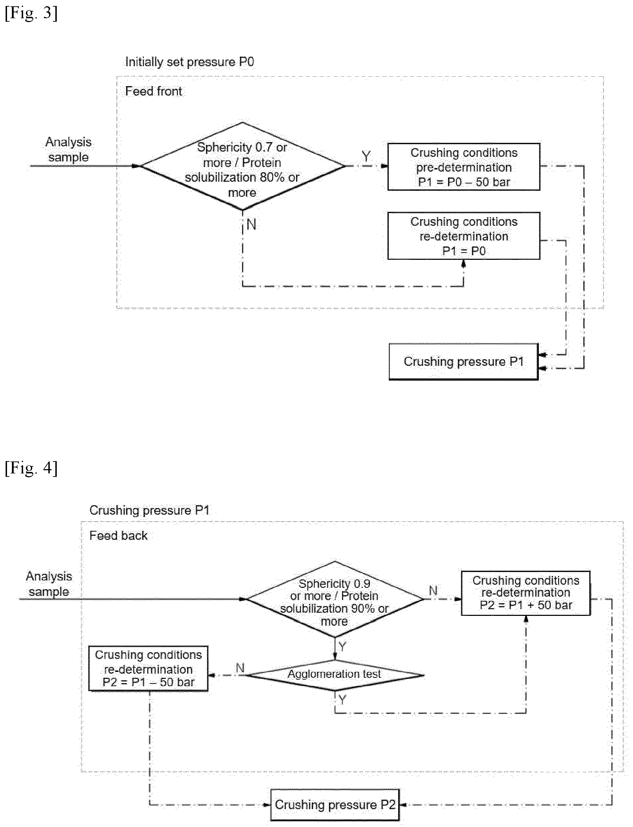

The method for controlling the crushing pressure in the second crushing conditions pre-determination step is specifically shown in Fig. 3.

Fig. 3 relates to the step of determining the crushing pressure (P1) in the step of crushing the cells or PHA contained in the feed solution with respect to the initially set pressure (P0) of the high-pressure homogenizer (second crushing conditions pre-determination step).

When the average sphericity of the cells or PHA contained in the feed solution is less than 0.7, or when the degree of protein solubilization is less than 80%, the crushing pressure (P1) in the step of crushing the cells or PHA contained in the feed solution is maintained to be the same as the initially set pressure (P0).

When the average sphericity of the cells or PHA contained in the feed solution is 0.7 or more, or when the degree of protein solubilization is 80% or more, the crushing pressure (P1) in the step of crushing the cells or PHA contained in the feed solution is reduced by 10 to 100 bar, for example, 50 bar, from the initially set pressure (P0).

**Crushing conditions re-determination step**

In another embodiment of the present invention, analyzing a morphological parameter of the cells or PHA contained in the crushed product solution is carried out during, or after, the crushing step, and the method may comprise quantifying the analyzed morphological parameter to detect the degree of crush; and determining the crushing pressure of the high-pressure homogenizer or the number of crushing treatments according to the degree of crush as a first re-determination step of crushing conditions.

In another embodiment of the present invention, a step of analyzing a morphological parameter of the cells or PHA contained in the crushed product solution is carried out during, or after, the crushing step, and the method may further comprise carrying out an agglomeration test to measure the degree of agglomeration of the crushed product solution after the step of quantifying the analyzed morphological parameter to detect the degree of crush; and a second pre-determination step of crushing conditions to determine the crushing pressure of the high-pressure homogenizer or the number of crushing treatments according to the degree of agglomeration. Each crushing conditions re-determination step is a separate step and is individually selected as needed. When the first re-determination step and the second re-determination step are adopted simultaneously in one process, the second re-determination step is carried out after the first re-determination step.

1) First crushing conditions re-determination step according to the analysis of a morphological parameter

A step of analyzing a morphological parameter of the cells or PHA contained in the crushed product solution is carried out during, or after, the crushing step, and the method may comprise a first crushing conditions re-determination step to quantify the analyzed morphological parameter to re-determine the crushing pressure of the high-pressure homogenizer or the number of crushing treatments for the feed solution continuously fed.

As an example, when the average sphericity of the cells or PHA contained in the crushed product solution is less than 0.9, the first crushing conditions re-determination step may raise the pressure by 10 to 100 bar from the crushing pressure in the step of crushing the cells or PHA contained in the feed solution.

2) First crushing conditions re-determination step according to the analysis of the degree of protein solubilization

As an example, when the degree of protein solubilization of the crushed product solution according to the above Equation 1 is less than 90%, the first crushing conditions re-determination step may raise the pressure by 10 to 100 bar from the crushing pressure in the step of crushing the cells or PHA contained in the feed solution.

The degree of crush can be measured through the average sphericity of the cells or PHA contained in the crushed product solution or the degree of protein solubilization thereof. Based on the measurement results, the crushing conditions may be adjusted: if the degree of crush is determined to be insufficient, the crushing pressure may be raised; or if the crush degree is determined to be excessive, the crushing pressure may be reduced.

3) Second crushing conditions re-determination step according to the degree of agglomeration

In another embodiment of the present invention, a step of analyzing a morphological parameter of the cells or PHA contained in the crushed product solution is carried out during, or after, the crushing step, and the method may comprise carrying out an agglomeration test to measure the degree of agglomeration of the crushed product solution; and a second crushing conditions re-determination step to determine the crushing pressure of the high-pressure homogenizer or the number of crushing treatments for the feed solution continuously fed according to the degree of agglomeration.

As an example, when the average sphericity of the PHA contained in the crushed product solution is 0.9 or more, or when the degree of protein solubilization is 90% or more, the agglomeration test is carried out. When the degree of agglomeration is measured to exceed a predetermined value, the second crushing conditions re-determination step may reduce the pressure by 10 to 100 bar from the crushing pressure in the step of crushing the cells or PHA contained in the feed solution.

As another example, when the degree of agglomeration is measured to be a predetermined value or less, the second crushing conditions re-determination step may maintain or raise the pressure by 10 to 100 bar from the crushing pressure in the step of crushing the cells or PHA contained in the feed solution.

If the degree of cell crush is high due to excessive physical crush in the crushing step, agglomeration of a PHA may occur. Therefore, piping clogging or the like can be prevented in the subsequent steps through the determination of crushing conditions in real time according to the morphological parameter analysis.

The method for controlling the crushing pressure in the crushing conditions re-determination step is specifically shown in Figs. 4 and 5.

Fig. 4 relates to the step of re-determining the crushing pressure (P2) of the high-pressure homogenizer for the feed solution continuously fed with respect to the crushing pressure (P1) in the step of crushing the cells or PHA contained in the feed solution.

A step of crushing the cells or PHA contained in the feed solution (P1); and an analysis step of analyzing a morphological parameter of the cells or PHA contained in the crushed product solution are carried out.

When the average sphericity of the cells or PHA contained in the crushed product solution is less than 0.9, or when the degree of protein solubilization is less than 90%, a pressure raised by 50 bar from the crushing pressure (P1) in the step of crushing the cells or PHA contained in the feed solution is determined to be the crushing pressure (P2) of the high-pressure homogenizer for the feed solution continuously fed.

When the average sphericity of the cells or PHA contained in the crushed product solution is 0.9 or more, or when the degree of protein solubilization is 90% or more, an agglomeration test to measure the degree of agglomeration in the crushed product solution is carried out.

When the degree of agglomeration is measured to be a predetermined value or less (Y), a pressure raised by 50 bar from the crushing pressure (P1) in the step of crushing the cells or PHA contained in the feed solution is determined to be the crushing pressure (P2) of the high-pressure homogenizer for the feed solution continuously fed.

When the degree of agglomeration is measured to exceed a predetermined value (N), a pressure reduced by 50 bar from the crushing pressure (P1) in the step of crushing the cells or PHA contained in the feed solution is determined to be the crushing pressure (P2) of the high-pressure homogenizer for the feed solution continuously fed.

Fig. 5 shows the crushing conditions re-determination step after the pressure is adjusted according to the agglomeration test in another embodiment of the present invention. It relates to the step of re-determining the crushing pressure (Pn+1) of the high-pressure homogenizer for the feed solution continuously fed after a crushed product solution is prepared at the crushing pressure (Pn) of the step of crushing the cells or PHA contained in the feed solution by increasing or reducing the pressure by 10 to 100 bar from the previously set crushing pressure (Pn-1).

Specifically, a step of crushing the cells or PHA contained in the feed solution at a crushing pressure (Pn) reduced by 50 bar from the previously set crushing pressure (Pn-1); and an analysis step of analyzing a morphological parameter of the cells or PHA contained in the crushed product solution are carried out.

Next, when the average sphericity of the cells or PHA contained in the crushed product solution is less than 0.9, or when the degree of protein solubilization is less than 90%, the first crushing conditions re-determination step determines a pressure to be determined between the previously set crushing pressure (Pn-1) and the crushing pressure (Pn) in the step of crushing the cells or PHA contained in the feed solution as the crushing pressure (Pn+1) of the high-pressure homogenizer for the feed solution continuously fed.

When the average sphericity of the cells or PHA contained in the crushed product solution is 0.9 or more, or when the degree of protein solubilization is 90% or more, an agglomeration test to measure the degree of agglomeration in the crushed product solution is carried out.

When the degree of agglomeration is measured to be a predetermined value or less (Y), the second crushing conditions re-determination step determines a pressure that is the same as the crushing pressure (Pn) in the step of crushing the cells or PHA contained in the feed solution as the crushing pressure (Pn+1) of the high-pressure homogenizer for the feed solution continuously fed.

When the degree of agglomeration is measured to exceed a predetermined value (N), the second crushing conditions re-determination step determines a pressure that is reduced by 50 bar from the crushing pressure (Pn) in the step of crushing the cells or PHA contained in the feed solution as the crushing pressure (Pn+1) of the high-pressure homogenizer for the feed solution continuously fed.

In the crushing conditions pre-determination step and the crushing conditions re-determination step, the criteria for determining the crushing conditions according to the morphological parameter, the degree of protein solubilization, or the degree of agglomeration of the cells or PHA may be adjusted according to the culture medium fed to the entire process.

**Embodiments for Carrying Out the Invention**

Hereinafter, the present invention will be described in more detail with reference to the following examples. But the following Examples are intended to illustrate the present invention, and the scope of the Examples is not limited thereto only.

**[Example]**

**1. Preparation of a culture medium**

A genetically engineered *E. coli* strain was used to prepare a PHA culture medium. First, the PHA-producing strain was seed-cultured to secure a certain amount, and main fermentation was then carried out. For the fermentation of the PHA-producing strain, sucrose was used as a carbon source, and various mineral components were added as nutrients. A sodium hydroxide solution or ammonia gas was used to adjust the fermentation pH. The fermentation process using the PHA-producing strain was carried out at 30 to 40°C and a pH of 6 to 9.

In Examples 1 and 2, separate culture media using a genetically engineered *E. coli* strain and sucrose as a carbon source were used. Although the culture media of Examples 1 and 2 were prepared under the same culture conditions, the morphological parameters of the cells or PHA measured in Example 1 or 2 may be different due to the nature of the strain as a living cell.

**2. Simulation of the autolysis of a culture medium**

The degree of autolysis refers to the degree to which cell walls weaken or cells burst due to autolysis. To simulate this experimentally, autolysis conditions were simulated using an ultrasonic disperser (SONICS, Vibra cell). A normally fermented culture was treated with an ultrasonic disperser at an amplitude of 40% and a pulse of 10/10 for 1 or 2 minutes to burst the cells to simulate the degree of autolysis, and a cell crushing process was then carried out.

In Examples 1-1 to 1-3, culture media treated with ultrasonic waves according to Table 1 below were used. Specifically, Example 1-1 was the culture medium of Example 1 without treatment with an ultrasonic disperser, and Example 1-2 or 1-3 were the culture media of Example 1 treated with an ultrasonic disperser for 1 minute or 2 minutes, respectively. Examples 2-1 to 2-3 were culture media treated with ultrasonic waves according to Table 2 below.

**3. Cell crushing process**

Each culture medium was subjected to the separation of impurities from the biomass containing the cells and/or PHA contained in the culture medium using a centrifuge (Hanil, Combi-514 R). The separated biomass was diluted using distilled water to have a total solids content of 10 to 15% by weight. The diluted solution was adjusted to a pH of 10 to 11 using sodium hydroxide (40 w/w%, Deoksan Science) and then added with sodium laureth sulfate (30 w/w%, Miwon), an anionic surfactant, at 0.13 to 0.15% by weight in the liquid phase to prepare a feed solution. The feed solution for crushing was sufficiently stirred and subjected to physical cell crushing using a high-pressure homogenizer (Bertoli, ATOMO 3.0) at 600 to 1,000 bar and 1 to 5 times, respectively.

**[Test Example]**

**1. Sample preparation for three-dimensional image analysis and measurement**

The analysis samples (culture medium, feed solution, or crushed product solution) of the Examples and Comparative Examples were each diluted 10 times or 100 times in a 1.5-ml microtube using distilled water (DIW). 20 $\mu$l of the diluted sample was dispensed into TomoDish (Tomo Cube Co., Ltd.) and covered with a cover slide. Three-dimensional images of the prepared sample were taken using holotomography analysis (Tomo Cube Co., Ltd., HT-1H) to measure cell length and sphericity.

The refractive index (RI) used for the three-dimensional image implementation and data measurement was 1.3513 to 1.3745 (for cells) and 1.3748 to 1.4081 (for PHA). Ten three-dimensional images were taken for each individual sample. TomoStudio™ (Tomo Cube Co., Ltd.) was used to implement three-dimensional images and analyze imaging sphericity. The average mass, volume, length, and sphericity of the cells and PHA were calculated.

**2. Measurement of protein content before and after cell crush**

The analysis samples (feed solution or crushed product solution) of the Examples and Comparative Examples were each crushed under the conditions of 300 to 1,500 bar at room temperature and then centrifuged at 15,000 rpm for 5 minutes using a microcentrifuge (Eppendorf, 5414R). The centrifuged supernatant was diluted 100 times in a 2.0-ml microtube with distilled water, and the absorbance was then measured at a wavelength of 280 nm using a UV-spectrometer (HACH, DR6000). When absorbance was measured, calibration was carried out with distilled water.

The degree of protein solubilization was calculated according to the following Equation 1.

[Equation 1]

$$\text{Degree of protein solubilization (\%)} = \text{ABS1/ABS0} \times 100 \ (\%)$$

In Equation 1, ABS0 is the absorbance at a wavelength of 280 nm measured after repeatedly crushing the analysis sample 5 times at 800 bar at room temperature; and ABS1 is the absorbance at a wavelength of 280 nm measured after crushing the analysis sample 1 time at 300 to 1,500 bar at room temperature.

**3. Agglomeration test**

50 ml of the analysis sample (lysate) was centrifuged at 3,800 rpm for 20 minutes (Hanil, Combi-514R), and the supernatant was removed. The retentate remaining at the bottom was passed through a 150-$\mu$m sieve with washing water. In such an event, the residue that was not dissolved in water and did not pass through the sieve was observed with the naked eye. Depending on the degree of remaining, the degree of agglomeration was classified into $\times$, $\Delta$, $\circ$, and $\circledcirc$ and shown in Tables 3 and 4 below. If the degree of agglomeration is $\times$ or $\Delta$, it was determined to be below the predetermined value (Y in Figs. 4 and 5).

**1) Results and analysis of the simulation of autolysis**

The analysis results of length and sphericity of the cells in the culture media of Examples 1-1 to 1-3 and 2-1 to 2-3 are summarized in Tables 1 and 2 below. To confirm reproducibility, the experiment was conducted twice with different culture media. As a result, as the time for ultrasonic disperser treatment increased, the average length of the cells in the culture medium decreased, and the average sphericity of the cells increased.

[Table 1]

| Example 1 | Ex. 1-1 | Ex. 1-2 | Ex. 1-3 |
|---|---|---|---|
| Ultrasonic treatment | None | 1 minute | 2 minutes |
| Average cell length ($\mu$m) | 4.92 | 4.74 | 4.65 |
| Cell sphericity (1-0.8)% | 11.81 | 18.66 | 15.30 |
| Cell sphericity (0.8-0)% | 88.19 | 81.34 | 84.70 |
| Average cell sphericity | 0.59 | 0.61 | 0.64 |
| PHA sphericity (1-0.8)% | 23.09 | 34.86 | 38.59 |
| PHA sphericity (0.8-0)% | 76.91 | 65.14 | 61.41 |
| Average PHA sphericity | 0.67 | 0.70 | 0.72 |

[Table 2]

| Example 2 | Ex. 2-1 | Ex. 2-2 | Ex. 2-3 |
|---|---|---|---|
| Ultrasonic treatment | None | 1 minute | 2 minutes |
| Average cell length ($\mu$m) | 4.01 | 3.56 | 3.34 |
| Cell sphericity (1-0.8)% | 39.81 | 53.38 | 58.86 |
| Cell sphericity (0.8-0)% | 60.19 | 46.62 | 41.15 |
| Average cell sphericity | 0.76 | 0.79 | 0.83 |
| PHA sphericity (1-0.8)% | 45.16 | 61.39 | 50.58 |
| PHA sphericity (0.8-0)% | 54.84 | 38.61 | 49.42 |

(continued)

| Example 2 | Ex. 2-1 | Ex. 2-2 | Ex. 2-3 |
|---|---|---|---|
| Average PHA sphericity | 0.78 | 0.84 | 0.82 |

**2) Analysis of the degree of cell crush**

The culture media of Examples 1 and 2 were each used to prepare a feed solution.

The morphological parameters, the degree of protein solubilization, and the degree of aggregation were measured for the cells and PHA of the feed solution at the feeding point of a high-pressure homogenizer; and the cells and PHA in the cell-crushed product solution after cell crush was carried out once at a crushing pressure of the high-pressure homogenizer of 600 to 1,000 bar. The results are shown in Tables 3 and 4 below.

[Table 3]

| | HPH crushing pressure (bar) | Avg. cell length (μm) | Cell sphericity | | Ave. cell sphericity | PHA sphericity | | Avg. PHA sphericity | Degree of agglomeration | Degree of protein solubilization (%) |
|---|---|---|---|---|---|---|---|---|---|---|
| | | | 1-0.8% | 0.8-0% | | 1-0.8% | 0.8-0% | | | |
| Ex. 1-1 | Feeding | 4.50 | 11.19 | 88.81 | 0.63 | 31.53 | 68.47 | 0.67 | × | 81.3 |
| | 600 | 4.26 | 35.34 | 64.66 | 0.72 | 59.93 | 40.07 | 0.83 | × | 97.4 |
| | 800 | 4.41 | 34.09 | 65.91 | 0.68 | 64.38 | 35.63 | 0.83 | × | 102.4 |
| | 1000 | 4.02 | 43.16 | 56.84 | 0.75 | 65.19 | 34.81 | 0.84 | × | 101.1 |
| Ex. 1-2 | Feeding | 4.71 | 13.39 | 86.61 | 0.63 | 36.92 | 63.08 | 0.71 | × | 75.6 |
| | 600 | 4.17 | 28.48 | 71.52 | 0.68 | 64.91 | 35.10 | 0.82 | △ | 93.3 |
| | 800 | 3.95 | 34.59 | 65.41 | 0.70 | 61.43 | 38.57 | 0.83 | △ | 96.3 |
| | 1000 | 3.83 | 41.35 | 58.65 | 0.74 | 69.57 | 30.43 | 0.85 | ○ | 92.9 |
| Ex. 1-3 | Feeding | 4.32 | 27.34 | 72.66 | 0.70 | 47.99 | 52.01 | 0.73 | × | 70.0 |
| | 600 | 4.03 | 39.35 | 60.65 | 0.73 | 52.81 | 47.19 | 0.81 | ○ | 82.1 |
| | 800 | 4.04 | 35.90 | 64.10 | 0.72 | 65.77 | 34.23 | 0.84 | ○ | 86.6 |
| | 1000 | 3.88 | 33.70 | 66.30 | 0.71 | 65.79 | 34.21 | 0.84 | ◎ | 86.4 |

[Table 4]

| | HPH crushing pressure (bar) | Avg. cell length (μm) | Cell sphericity | | Avg. cell sphericity | PHA sphericity | | Avg. PHA sphericity | Degree of agglomeration | Degree of protein solubilization (%) |
|---|---|---|---|---|---|---|---|---|---|---|
| | | | 1-0.8% | 0.8-0% | | 1-0.8% | 0.8-0% | | | |
| Ex. 2-1 | Feeding | 3.82 | 47.28 | 52.72 | 0.79 | 63.09 | 36.91 | 0.84 | △ | 2.8 |
| | 600 | 3.61 | 56.80 | 43.20 | 0.81 | 80.09 | 19.91 | 0.90 | ◎ | 91.9 |
| | 800 | 3.27 | 71.06 | 28.94 | 0.85 | 86.60 | 13.40 | 0.93 | ◎ | 93.3 |
| | 1000 | 3.23 | 62.66 | 37.34 | 0.83 | 84.72 | 15.29 | 0.92 | ◎ | 144.4 |
| Ex. 2-2 | Feeding | 3.58 | 49.02 | 50.98 | 0.79 | 60.22 | 39.78 | 0.84 | △ | 35.6 |
| | 600 | 3.31 | 63.16 | 36.84 | 0.85 | 84.71 | 15.29 | 0.93 | ◎ | 71.1 |
| | 800 | 3.62 | 47.41 | 52.59 | 0.80 | 78.10 | 21.90 | 0.90 | ◎ | 89.6 |
| | 1000 | 3.16 | 60.69 | 39.31 | 0.84 | 82.29 | 17.71 | 0.90 | ◎ | 143.0 |
| Ex. 2-3 | Feeding | 3.31 | 58.64 | 41.36 | 0.83 | 76.98 | 23.02 | 0.88 | ○ | 23.0 |
| | 600 | 3.07 | 60.95 | 39.05 | 0.83 | 76.23 | 23.77 | 0.89 | ◎ | 48.1 |
| | 800 | 3.35 | 58.96 | 41.04 | 0.82 | 79.46 | 20.54 | 0.91 | ◎ | 111.9 |
| | 1000 | 3.72 | 44.46 | 55.55 | 0.78 | 78.00 | 22.00 | 0.90 | ◎ | 123.7 |

As a result of the analysis, it was found that the tendency of the degree of agglomeration varied depending on the degree of autolysis of the culture medium and the crushing pressure of the crushing step.

Specifically, when the average cell length of the feed solution or crushed product solution was 4.00 $\mu$m or more, and when the average cell sphericity of the feed solution or the average PHA sphericity of the crushed product solution was 0.85 or less, the degree of agglomeration was hardly observed, resulting in high process stability in the downstream process.

When the average cell length of the feed solution or crushed product solution was 3.28 to 4.20 $\mu$m, and when the average cell sphericity of the feed solution or the average PHA sphericity of the crushed product solution was 0.75 to 0.85, the degree of agglomeration was slightly observed, resulting in a slight decrease in the process stability in the downstream process; however, ordinary process operation would be possible.

When the average cell length of the feed solution or crushed product solution was 3.92 $\mu$m or less, and when the average cell sphericity of the feed solution or the average PHA sphericity of the crushed product solution was 0.83 to 1.00, a degree of agglomeration at a significant or high level was observed, resulting in a significant decrease in the process stability in the downstream process and a probable problem such as piping clogging or the like.

In addition, when the number of cells with a cell sphericity of 0 to 0.8 was 50.0% or more of the total, the degree of agglomeration was observed at a low level or almost not observed. In particular, when it was 70.0% or more, the degree of agglomeration was hardly observed.

In Example 1 or 2, the degree of protein solubilization was calculated based on the ABSO of Example 1-1 or Example 2-1, which was a culture medium not subjected to ultrasonic treatment, respectively. When cells were crushed with a high-pressure homogenizer at a crushing pressure of 600 bar or higher, protein solubilization was 90% or more in Examples 1-1, 1-2, and 2-1, whereas the protein solubilization was less than 90% in Examples 1-3, 2-2, and 2-3 in which the degree of autolysis was high. It is understood that, in a culture medium subjected to ultrasonic treatment, a relatively large amount of protein was separated during the process of preparing a feed solution containing the supernatant obtained by centrifuging the culture medium; thus, the residual protein content was lower than that of the culture medium not subjected to ultrasonic treatment.

In Examples 2-1 to 2-3, the degree of protein solubilization exceeding 100% was attributable to an error due to the absorbance measurement method, and this should be understood to mean that the degree of protein solubilization was 90% or more.

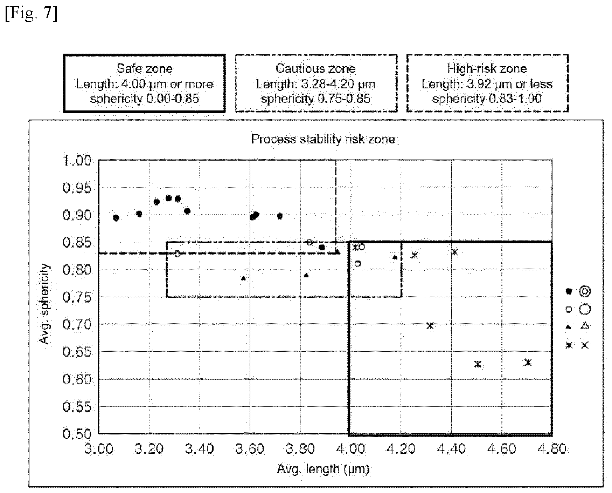

Based on the above results, the ranges of length (average cell length or average PHA length) and sphericity (average cell sphericity or average PHA sphericity) of cells or PHA contained in the feed solution to determine process stability were classified into three zones and shown in Fig. 7.

1. Safe zone: length 4.00 $\mu$m or more and sphericity 0.00-0.85
2. Cautious zone: length 3.28-4.20 $\mu$m and sphericity 0.75-0.85
3. High-risk zone: length 3.92 $\mu$m or less and sphericity 0.83-1.00.

However, Fig. 7 is an example prepared based on the culture media of Examples 1 and 2 during fermentation, and the culture media tend to agglomerate together relatively well; thus, the range of the cautious zone and safe zone of length and sphericity appears relatively narrow in Fig. 7. The value of each zone may vary as the strain or type of carbon source in the culture medium changes.

**Claims**

1. A method for monitoring cell crush, wherein the degree of cell crush is confirmed, or the crushing conditions are determined by analyzing a morphological parameter of cells or a polyhydroxyalkanoate (PHA) contained in a culture medium containing the PHA through three-dimensional image analysis.

2. The method for monitoring cell crush of claim 1, wherein the three-dimensional image analysis is a three-dimensional image analysis using holotomography analysis, and the morphological parameter is one or more selected from the group consisting of length, sphericity, volume, and surface area.

3. A method for producing a polyhydroxyalkanoate (PHA), which comprises:

   preparing a culture medium containing a polyhydroxyalkanoate (PHA);
   preparing a feed solution containing the culture medium;
   crushing the cells or PHA contained in the feed solution; and
   analyzing a morphological parameter of the cells or the PHA before, during, or after the crushing step.

4. The method for producing a polyhydroxyalkanoate (PHA) of claim 3, wherein analyzing a morphological parameter of the cells or the PHA contained in the culture medium is carried out before the crushing step, and
the method comprises quantifying the analyzed morphological parameter to detect a degree of autolysis; and determining the initially set pressure of the high-pressure homogenizer or the number of crushing treatments according to the degree of autolysis as a first pre-determination step of crushing conditions.

5. The method for producing a polyhydroxyalkanoate (PHA) of claim 3, wherein analyzing a morphological parameter of the cells or the PHA contained in the feed solution is carried out before the crushing step, and
the method comprises quantifying the analyzed morphological parameter to detect a feed status; and determining the crushing pressure of the high-pressure homogenizer or the number of crushing treatments according to the feed status as a second pre-determination step of crushing conditions.

6. The method for producing a polyhydroxyalkanoate (PHA) of claim 3, wherein analyzing a morphological parameter of the cells or the PHA contained in the crushed product solution is carried out during, or after, the crushing step, and
the method comprises quantifying the analyzed morphological parameter to detect a degree of crush; and determining the crushing pressure of the high-pressure homogenizer or the number of crushing treatments according to the degree of crush as a first re-determination step of crushing conditions.

7. The method for producing a polyhydroxyalkanoate (PHA) of claim 3, wherein the crushing pressure in the crushing step is 300 to 1,500 bar.

8. The method for producing a polyhydroxyalkanoate (PHA) of claim 3, which comprises a first analysis step of analyzing a morphological parameter of the cells or the PHA contained in the feed solution;

a second pre-determination step of crushing conditions to determine the crushing pressure of the high-pressure homogenizer or the number of crushing treatments according to the first analysis results;
a step of crushing the cells or the PHA contained in the feed solution;
a second analysis step of analyzing a morphological parameter of the cells or the PHA contained in the crushed product solution; and
a first re-determination step of crushing conditions to determine the crushing pressure of the high-pressure homogenizer or the number of crushing treatments according to the second analysis results.

9. The method for producing a polyhydroxyalkanoate (PHA) of any one of claims 3 to 8, wherein the degree of protein solubilization according to the following Equation 1 is 75 to 110%:

[Equation 1]

$$\text{Degree of protein solubilization (\%)} = ABS1/ABS0 \times 100\ (\%)$$

in Equation 1, ABS0 is the absorbance at a wavelength of 280 nm measured after 5 times repeatedly crushing the culture medium, feed solution, or crushed product solution at 800 bar at room temperature; and ABS 1 is the absorbance at a wavelength of 280 nm measured after 1 time crushing the culture medium, feed solution, or crushed product solution at 300 to 1,500 bar at room temperature.

10. The method for producing a polyhydroxyalkanoate (PHA) of claim 5, wherein, when the average sphericity of the cells or the PHA contained in the feed solution is 0.7 or more, or when the degree of protein solubilization according to the following Equation 1 is 80% or more, in the second pre-determination step of crushing conditions the pressure is reduced by 10 to 100 bar from the initial set pressure in the step of crushing the cells or the PHA contained in the feed solution:

[Equation 1]

$$\text{Degree of protein solubilization (\%)} = ABS1/ABS0 \times 100\ (\%)$$

in Equation 1, ABS0 is the absorbance at a wavelength of 280 nm measured after 5 times repeatedly crushing the culture medium, feed solution, or crushed product solution at 800 bar at room temperature; and ABS1 is the absorbance at a wavelength of 280 nm measured after 1 time crushing the culture medium, feed solution, or crushed product solution at 300 to 1,500 bar at room temperature.

11. The method for producing a polyhydroxyalkanoate (PHA) of claim 6, wherein, when the average sphericity of the cells or the PHA contained in the crushed product solution is less than 0.9, or when the degree of protein solubilization according to the following Equation 1 is less than 90%, in the first re-determination step of crushing conditions the pressure is raised by 10 to 100 bar from the crushing pressure in the step of crushing the cells or the PHA contained in the feed solution:

[Equation 1]

Degree of protein solubilization (%) = ABS1/ABS0 × 100 (%)

in Equation 1, ABS0 is the absorbance at a wavelength of 280 nm measured after 5 times repeatedly crushing the culture medium, feed solution, or crushed product solution at 800 bar at room temperature; and ABS 1 is the absorbance at a wavelength of 280 nm measured after 1 time crushing the culture medium, feed solution, or crushed product solution at 300 to 1,500 bar at room temperature.

12. The method for producing a polyhydroxyalkanoate (PHA) of claim 8, which further comprises carrying out an agglomeration test to measure the degree of agglomeration of the crushed product solution after the second analysis step is carried out; and as a second re-determination step of crushing conditions determining the crushing pressure of the high-pressure homogenizer or the number of crushing treatments according to the degree of agglomeration.

13. A system for monitoring cell crush in which the degree of cell crush is confirmed, or the crushing conditions are determined by analyzing a morphological parameter of cells or a polyhydroxyalkanoate (PHA) contained in a culture medium containing the PHA through three-dimensional image analysis.

[Fig. 1]

[Fig. 2]

[Fig. 3]

Initially set pressure P0

Feed front

Analysis sample → ⟨Sphericity 0.7 or more / Protein solubilization 80% or more⟩ —Y→ Crushing conditions pre-determination P1 = P0 – 50 bar

N

Crushing conditions re-determination P1 = P0

Crushing pressure P1

[Fig. 4]

Crushing pressure P1

Feed back

Analysis sample → ⟨Sphericity 0.9 or more / Protein solubilization 90% or more⟩ —N→ Crushing conditions re-determination P2 = P1 + 50 bar

Y

Crushing conditions re-determination P2 = P1 – 50 bar ←N— ⟨Agglomeration test⟩

Y

Crushing pressure P2

[Fig. 5]

[Fig. 6]

[Fig. 7]

## INTERNATIONAL SEARCH REPORT

| | International application No. |
|---|---|
| | **PCT/KR2022/021206** |

### A. CLASSIFICATION OF SUBJECT MATTER

**G01N 33/483**(2006.01)i; **G01N 21/31**(2006.01)i; **C12P 7/62**(2006.01)i; **G01N 21/17**(2006.01)i

According to International Patent Classification (IPC) or to both national classification and IPC

### B. FIELDS SEARCHED

Minimum documentation searched (classification system followed by classification symbols)

G01N 33/483(2006.01); A61K 51/06(2006.01); A61K 9/08(2006.01); C12P 7/62(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 세포 파쇄 모니터링(cell disrupture monitoring), 폴리하이드록시알카노에이트 (PHA), 3차원 이미지 분석(3D image analysis), 고압 균질기(high pressure homogenizer), 파쇄 조건 선결정(disrupting parameter pre-determination), 파쇄 조건 재결정(disrupting parameter re-determination)

### C. DOCUMENTS CONSIDERED TO BE RELEVANT

| Category* | Citation of document, with indication, where appropriate, of the relevant passages | Relevant to claim No. |
|---|---|---|
| X<br>Y | HWANG, Kuo-Jen et al. Disruption kinetics of bacterial cells during purification of poly-β-hydroxyalkanoate using ultrasonication. Journal of the Chinese Institute of Chemical Engineers. 2006, vol. 37, no. 3, pp. 1-8.<br>See abstract; and pages 1 and 3-4. | 1,3-8,12-13<br>2,9-11 |
| Y | CHOI, So Young et al. Three-dimensional label-free visualization and quantification of polyhydroxyalkanoates in individual bacterial cell in its native state. PNAS. 26 July 2021, vol. 118, no. 31, article no. e2103956118, pp. 1-8.<br>See abstract; and pages 2 and 7-8. | 2 |
| Y | MOJAVERYAZDI, Farzaneh Sabbagh et al. Importance of glucose and Pseudomonas to produce degradable plastics. PPI-UTM Technology, Education, and Science International Conference (TESIC) 2013. 2013, pp. 1-6. Retrieved from <DOI: https://doi.org/10.11113/jt.v69.3194>.<br>See abstract; and page 3. | 9-11 |
| A | JP 2018-528235 A (SERENE, LLC) 27 September 2018 (2018-09-27)<br>See entire document. | 1-13 |

☑ Further documents are listed in the continuation of Box C.　　☑ See patent family annex.

| | |
|---|---|
| *　Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **28 March 2023** | **29 March 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2022/021206** |

## C. DOCUMENTS CONSIDERED TO BE RELEVANT

| Category* | Citation of document, with indication, where appropriate, of the relevant passages | Relevant to claim No. |
| --- | --- | --- |
| A | US 2012-0252081 A1 (YU, Jian) 04 October 2012 (2012-10-04)<br>See entire document. | 1-13 |

Form PCT/ISA/210 (second sheet) (July 2022)

# EP 4 455 662 A1

| INTERNATIONAL SEARCH REPORT<br>Information on patent family members | | | International application No.<br>**PCT/KR2022/021206** | |

| Patent document<br>cited in search report | | | Publication date<br>(day/month/year) | Patent family member(s) | | | Publication date<br>(day/month/year) |
|---|---|---|---|---|---|---|---|
| JP | 2018-528235 | A | 27 September 2018 | CA | 2999975 | A1 | 30 March 2017 |
| | | | | CN | 108348619 | A | 31 July 2018 |
| | | | | EP | 3352797 | A1 | 01 August 2018 |
| | | | | KR | 10-2018-0083304 | A | 20 July 2018 |
| | | | | US | 2019-0134238 | A1 | 09 May 2019 |
| | | | | WO | 2017-053141 | A1 | 30 March 2017 |
| US | 2012-0252081 | A1 | 04 October 2012 | AU | 2009-354139 | A1 | 10 May 2012 |
| | | | | AU | 2009-354139 | B2 | 27 November 2014 |
| | | | | BR | 112012008850 | A2 | 25 July 2017 |
| | | | | BR | 112012008850 | B1 | 21 November 2018 |
| | | | | DK | 2488653 | T3 | 09 December 2013 |
| | | | | EP | 2488653 | A1 | 22 August 2012 |
| | | | | EP | 2488653 | B1 | 28 August 2013 |
| | | | | ES | 2437340 | T3 | 10 January 2014 |
| | | | | HR | P20131138 | T1 | 17 January 2014 |
| | | | | JP | 2013-507908 | A | 07 March 2013 |
| | | | | JP | 5735970 | B2 | 17 June 2015 |
| | | | | PL | 2488653 | T3 | 31 March 2014 |
| | | | | PT | 2488653 | E | 02 December 2013 |
| | | | | US | 2017-0002385 | A1 | 05 January 2017 |
| | | | | WO | 2011-045625 | A1 | 21 April 2011 |
| | | | | ZA | 201202607 | B | 26 June 2013 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BRITTA, EGGENREICH.** A combination of HPLC and automated data analysis for monitoring the efficiency of high-pressure homogenization. *Microbial Cell Factories,* 2017, vol. 16